# EUROPEAN PATENT APPLICATION

(11) **EP 4 218 835 A2**
(43) Date of publication of application: **02.08.2023**
(21) Application number: 23151907.5
(22) Date of filing: 17.01.2023
(51) Int. Cl.: A61L 2/10, A61L 2/14, A61L 9/22, B66B 31/00

(54) **STERILIZATION DEVICE**

(30) Priority: 28.01.2022 CN 202220238712 U
(71) Applicant: Shenzhen Envicool Health Environment Technology Co., Ltd, Shenzhen (CN)
(72) Inventor: YANG, Fan, Shenzhen (CN); WU, Jianming, Shenzhen (CN)
(74) Representative: Cabinet Chaillot

(57) **Abstract**

The application relates to the technical field of sterilization, and discloses a sterilization device. The sterilization device comprises a shell (1), an air guide passage (2), a sterilization module (3) and a blowing device (4). An opening (121) is formed in a back side of the shell (1) to allow a handrail (101) to pass therethrough. The air guide passage (2) is disposed in the shell (1) and has an air inlet (21) communicated with an ambient space outside the shell (1), as well as an air outlet (22) facing and communicating with the opening (121). The sterilization module (3) includes a plasma generator (31) disposed in the air guide passage (2) and capable of ionizing air and generating high-energy particles. The blowing device (4) is configured to force air outside the shell (1) into the air guide passage (2) and blow the ionized air and the high-energy particles out of the air outlet (22) of the air guide passage (2) to the handrail (101) passing through the opening (121). The sterilization device can accurately sterilize the handrail (101), thus having a good sterilization effect; and the sterilization device realizes sterilization based on air ionization, so the cost is low, and a pungent odor is avoided, which brings good experience to passengers riding an escalator.

## Description

### FIELD

The invention relates to the technical field of sterilization, in particular to a sterilization device.

### BACKGROUND

With the increase of the application demand for sterilization, sterilization is needed in many scenarios such as air, water, food, hospitals, public transport, hotels, and schools. For example, escalators which have been widely used in public places with a large volume of people such as shopping malls, hospitals and trains need to be sterilized because the handrail of the escalators are quite likely be contaminated with bacteria or viruses when touched by passengers who ride the escalators, which may in turn cause cross infection.

In the prior art, the handrail of the escalators is often sterilized with disinfectants. However, on the one hand, the disinfectants are consumables, so high disinfectant consumption and high costs will be caused by frequent sterilization; on the other hand, the disinfectants have a strong pungent smell, which may affect the experience of passengers.

Thus, a sterilization device is urgently needed to solve the aforementioned problems.

### SUMMARY

The technical issue that the present invention aims to resolve is to provide an improved sterilization device which has a good sterilization effect without pungent odor being produced.

The technical solution that the present invention adopts to resolve the technical issue is to provide a sterilization device which comprises a shell having a side provided with an opening configured to face and receive a handrail of an escalator; an air guide passage disposed in the shell, the air guide passage comprising an air inlet communicated with an ambient space outside the shell, and an air outlet oriented to and communicated with the opening; a sterilization module comprising a plasma generator disposed in the air guide passage, the plasma generator being configured for ionizing air and generating high-energy particles; and a blowing device configured for forcing air outside the shell into the air guide passage and blowing ionized air and the high-energy particles to the opening via the air outlet of the air guide passage.

Optionally, the blowing device is disposed in the air guide passage and is located upstream of the plasma generator.

Optionally, the shell is provided with an air inlet opening, a grating is disposed at the air inlet opening, the ambient space outside the shell and an inside space within the shell are communicated with each other through the grating, and the air inlet of the air guide passage is communicated with the inside space within the shell.

Optionally, a filter module is disposed at the air inlet of the air guide passage.

Optionally, the sterilization module further comprises an ultraviolet sterilization lamp disposed in the shell, and the ultraviolet sterilization lamp faces the opening and is configured to emit ultraviolet light to the handrail through the opening.

Optionally, the sterilization device further comprises a wheel rotatably disposed at the opening and configured to abut against the handrail and be rotated under action of the handrail); a generator in drive connection with the wheel and configured to receive energy of motion from the wheel and transfer the energy of motion to electric energy; and an energy storage module configured for storing the electric energy generated by the generator and supplying power to the blowing device and/or the sterilization module.

Optionally, the sterilization device further comprises a power interface, wherein the power interface is electrically connected to the blowing device and the sterilization module and is configured to be connected to an external power source.

Optionally, the sterilization device further comprises a power supply mode change-over switch, wherein the power supply mode change-over switch is configured to select one of the power interface and the energy storage module to supply power to the blowing device and the sterilization module.

Optionally, the sterilization device further comprises an infrared detection module disposed on the shell, wherein the infrared detection module is configured to enable the blowing device and the sterilization module to work when detecting that a passenger enters the escalator.

Optionally, the infrared detection module is disposed at another side of the shell which is opposite to the side in which the opening is defined.

Optionally, the sterilization device has a mandatory working mode in which the blowing device and the sterilization module are manually controlled to work, as well as an intelligent working mode in which the blowing device and the sterilization module are controlled by the infrared detection module to work. Optionally, the sterilization device further comprises a working mode change-over switch configured to switch the mandatory working mode and the intelligent working mode.

Optionally, the sterilization device further comprises a display screen disposed on the shell.

Optionally, the sterilization device further comprises one or more indicator lights disposed on the shell.

Optionally, the opening is elongated and defines a lengthwise direction and a depth direction so that the handrail of the escalator can move in the opening along the lengthwise direction, the lengthwise direction and the depth direction both being slanted relative to a supporting surface on which the sterilization device stands.

Optionally, the air outlet has an orientation parallel to the depth direction of the opening.

The invention has at least the following beneficial effects:

According to the sterilization device provided by the invention, the air outlet of the air guide passage faces the opening in the shell and is thus oriented to the handrail of the escalator, the plasma generator located in the air guide passage can ionize air and generates a high-energy strong oxidizing group (high-energy particles) which can be blown onto the handrail under the action of the blowing device and react with nucleic acid substances of bacteria/viruses on the handrail to modify the nucleic acid substances so as to kill the bacteria/viruses, and the high-energy particles can break through bacterial cells and virus particles to kill the bacterial cells and virus particles. The sterilization device can accurately sterilize the handrail, thus having a good sterilization effect; and the sterilization device realizes sterilization based on air ionization, so the cost is low, and a pungent odor is avoided, which brings good experience to passengers riding the escalator.

### BRIEF DESCRIPTION OF THE DRAWINGS

FIG. 1 is a schematic structural view from one perspective of a sterilization device provided by one specific embodiment of the invention;
FIG. 2 is a schematic structural view from another perspective of the sterilization device provided by one specific embodiment of the invention;
FIG. 3 is an exploded view of the sterilization device provided by one specific embodiment of the invention;
FIG. 4 is a schematic internal structural view of the sterilization device provided by one specific embodiment of the invention;
FIG. 5 is a schematic structural view showing the sterilization device installed on an escalator according to one specific embodiment of the invention; and
FIG. 6 illustrates an electrical connection between a generator and an energy storage module of the sterilization device according to one specific embodiment of the invention.

In the figures:
100-escalator; 101-handrail;
1 -shell; 11-front shell; 12-back cover; 121-opening; 13-grating;
2-air guide passage; 21-air inlet; 22-air outlet;
3-sterilization module; 31-plasma generator; 32-ultraviolet sterilization lamp;
41-blowing device; 42-filter module;
51-wheel; 52-generator; 54-energy storage module;
61-power interface; 62-power supply mode change-over switch; 63-working mode change-over switch;
71-infrared detection module; 72-display screen; 73-indicator light;
8-main control circuit board.

### DESCRIPTION OF THE EMBODIMENTS

The invention will be described in further detail below in conjunction with the accompanying drawings and embodiments. It can be understood that the specific embodiments in the following description are merely used to explain the invention, and are not used to limit the invention. In addition, it should be noted that, for the convenience of description, only those parts relating to the invention rather than all structures are shown in the drawings.

In the description of the invention, unless otherwise specifically stated and defined, terms such as "connection", "connect" and "fix" should be broadly understood, which, for example, may refer to fixed connection, detachable connection or integrated connection; or, mechanical connection or electrical connection; or, direct connection, indirect connection through an intermediate medium, or internal communication or interaction of two elements. Those ordinarily skilled in the art can appreciate the specific meanings of these terms in the invention as the case may be.

In the invention, unless otherwise specifically stated and defined, the expression that a first feature is located "above" or "below" a second feature means that the first feature and the second feature are in direct contact or that the first feature and the second feature are in contact through another feature therebetween. Moreover, the expression that the first feature is located "above" or "over" the second feature means that the first feature is located exactly or obliquely above or over the second feature or only indicates that the first feature is higher than the second feature. The expression that the first feature is located "below" or "under" the second feature means that the first feature is located exactly or obliquely below or under the second feature or only indicates that the first feature is lower than the second feature.

In the description of the embodiments of the invention, terms such as "upper", "lower" and "right" are used to indicate directional or positional relationships based on the accompanying drawings merely for the purpose of facilitating and simplifying operation, do not indicate or imply a device or an element referred to must be in a specific direction, or be configured and operated in a specific direction, and thus should not be construed as limitations of the invention. In addition, terms such as "first" and "second" are merely used for a distinguishing purpose in the description, and have no special meaning.

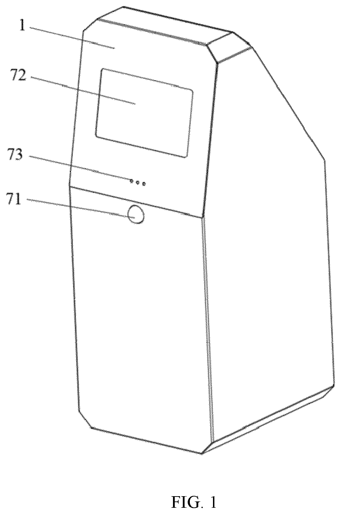

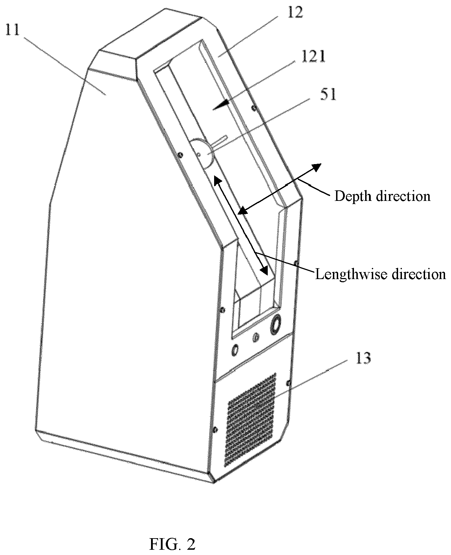

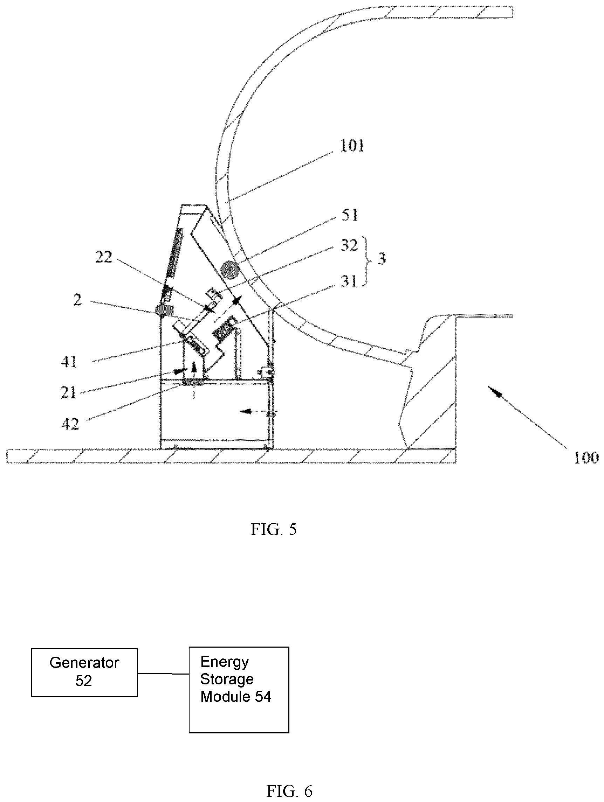

As shown in FIG. 1-FIG. 6, this embodiment provides a sterilization device which can be used for sterilizing a handrail 101 of an escalator 100. As shown in FIG. 5, the sterilization device is installed at an entrance of the escalator 100 during use. Specifically, as shown in FIG. 1 and FIG. 2, the sterilization device for the escalator 100 comprises a shell 1, wherein an opening 121 is defined in a back side (a side facing the escalator 100) of the shell 1, and the opening 121 faces the handrail 101 of the escalator 100 to allow the handrail 101 to run therethrough. Preferably, the opening 121 is elongated, and the elongated opening 121 matches the handrail 101 in shape, such that the portion of the handrail 101 received in the opening 121 can be partially encircled and covered by the shell 1. During the running process of the escalator 100, different portions of the handrail 101 sequentially passes through the opening 121 of the shell 1 with the rotation of the handrail 101, such that the handrail 101 can be sterilized by the sterilization device.

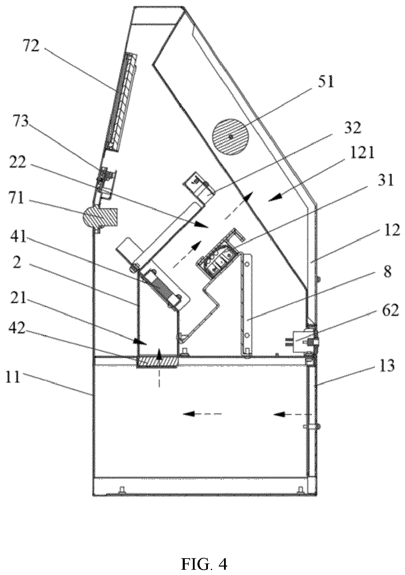

Specifically, as shown in FIG. 4 and FIG. 5 in which the dotted arrow indicates the flow direction of air in the sterilization device, the sterilization device further comprises an air guide passage 2, a sterilization module 3 and a blowing device 41. The air guide passage 2 is disposed in the shell 1, an air inlet 21 of the air guide passage 2 is communicated with an ambient space outside the shell 1, an air outlet 22 of the air guide passage 22 is oriented to the opening 121 and the handrail 101. The air outlet 22 is communicated with the opening 121. For example, the bottom wall of the opening 121 defines one or more holes or slots to communicate the air outlet 22 with the opening 121. The sterilization module 3 comprises a plasma generator 31 disposed in the air guide passage 2, and the plasma generator 31 is capable of ionizing air and generating high-energy particles. It should be noted that the plasma generator 31 may be any one type of plasma generator known in the art without significantly departing from the spirit of the invention. The plasma generator 31 can be selected and set according to actual requirements, and the invention has not specific limitation in this aspect. The blowing device 41 is capable of sucking/drawing air outside the shell 1 into the air guide passage 2 and blowing out the ionized air and the high-energy particles to the opening 121 from the air outlet 22 of the air guide passage 2.

According to the sterilization device provided by the invention, the air outlet 22 of the air guide passage 2 faces the opening 121 defined in the shell 1 and is thus oriented to the handrail 101 of the escalator 100, the plasma generator 31 located in the air guide passage 2 is configured to ionize air and generate high-energy strong oxidizing groups (high-energy particles). The high-energy strong oxidizing groups can be blown to the handrail 101 of the escalator 100 under the action of the blowing device 41 and react with nucleic acid substances of bacteria/viruses on the handrail 101 to modify the nucleic acid substances so as to kill the bacteria/viruses, and the high-energy particles can break through bacterial cells and virus particles to kill the bacterial cells and virus particles. The sterilization device can accurately sterilize the handrail 101, thus having a good sterilization effect; and the sterilization device realizes sterilization based on air ionization, so the cost is low, and a pungent odor is avoided, which brings good experience to passengers riding the escalator 100.

Specifically, in this embodiment, as shown in FIG. 1-FIG. 3, the shell 1 comprises a front shell 11 and a back cover 12 which can be assembled together, wherein a receiving space is defined by the front shell 11, the blowing device 41 of the air guide passage 2 is disposed in the receiving space, the back cover 12 covers the back side of the front shell 11, and the elongated opening 21 is formed in the back cover 12. Preferably, as shown in FIG. 4, the air guide passage 2 may be formed by sheet metal and is fixed in the shell 1, and the plasma generator 31 is disposed at an end, close to the opening 121 in the back cover 12, of the air guide passage 2, such that the blowing device 41 can blow all high-energy particles generated by the plasma generator 31 onto the handrail 101 of the escalator 100 through the opening 121. In other embodiments, the air guide passage 2 may be formed by other materials, and the invention has no limitations in this aspect.

Preferably, as shown in FIG. 4, the blowing device 41 is received in the air guide passage 2, and is located upstream of the plasma generator 31. It should be noted that, in this embodiment, "upstream" refers to a relatively front position in the flow direction of air in the air guide passage 2, that is, air flows through the blowing device 41 and then passes through the plasma generator 31. In this embodiment, the blowing device 41 is disposed in the shell 1 and is further located in the air guide passage 2, such that a larger air volume can be provided, and air carrying high-energy particles can fully contact the handrail 101, thus improving the sterilization effect; and noise generated during operation of the blowing device 41 can be greatly reduced, thus improving the use experience of users. In this embodiment, the blowing device 41 is disposed in the middle of the air guide passage 2.

Preferably, as shown in FIG. 2 and FIG. 4, an air inlet opening is formed in the shell 1, a grating 13 is disposed at the air inlet opening, the ambient space outside the shell 1 is communicated with an inside space within the shell 1 through the grating 13, and the air inlet 21 of the air guide passage 2 is communicated with the inside space within the shell 1, such that the air guide passage 2 is communicated with the ambient space outside the shell 1. The grading 13 can preliminarily filter outside air to prevent large debris and waste from entering the shell 1, and can also prevent non-operating personnel such as children from mistakenly stretching their hands into the shell 1, thus ensuring the safety of the sterilization device. In this embodiment, the air inlet opening is formed in the back cover 12, and the grating 13 is connected to the back cover 12 and is installed at the air inlet opening.

Further, as shown in FIG. 4, a filter module 42 is disposed at the air inlet 21 of the air guide passage 2. The filter module 42 can further filter air entering the shell 1 to remove fine dust in the air, such that dust is prevented from entering the blowing device 41 to reduce the service life of the blowing device 41. It should be noted that, the filter module 42 may be any type in the prior art without violating the concept of the application, and can be selected and set according to actual filter requirements, and the application has not specific limitation in this aspect.

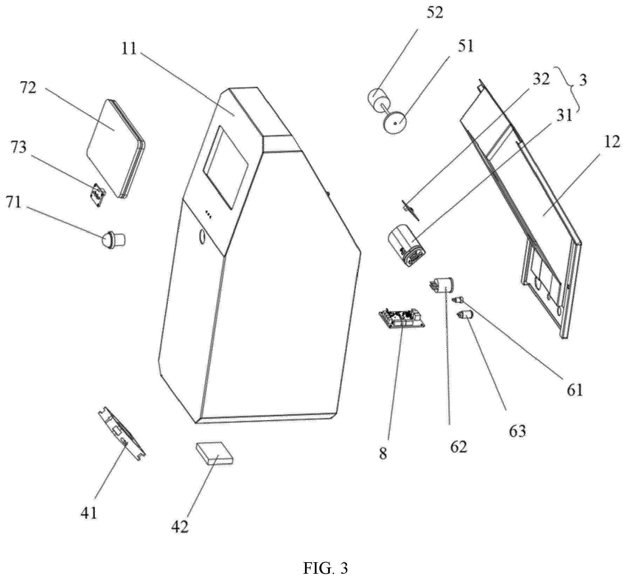

Preferably, as shown in FIG. 3 and FIG. 4, the sterilization module 3 further comprises an ultraviolet sterilization lamp 32 disposed in the shell 1, wherein the ultraviolet sterilization lamp 32 faces the opening 121 and the handrail 101. The ultraviolet sterilization lamp 32 can emit ultraviolet light to the handrail 101 through the opening 121 in the shell 1 to further sterilize the handrail 101, and dual sterilization of the handrail 101 is realized through the cooperation of the ultraviolet sterilization lamp 32 and the plasma generator, such that a good sterilization effect is achieved. In this embodiment, the ultraviolet sterilization lamp 32 is fixed at an outlet opening of the air guide passage 2 through a support member. Optionally, the ultraviolet sterilization lamp 32 may be a UVC ultraviolet sterilization lamp 32 with a wavelength ranging from 250 nm to 270 nm, and the sterilization effect of ultraviolet light in this waveband is optimal. In other embodiment, the ultraviolet sterilization lamp 32 may be a UVA ultraviolet sterilization lamp 32, or a combination of the UVC ultraviolet sterilization lamp 32 and the UVA ultraviolet sterilization lamp 32. The invention has no limitation in this aspect.

Preferably, as shown in FIG. 3 and FIG. 4, the sterilization device further comprises a main control circuit board 8 used for controlling on-off of the blowing device 41, the sterilization module 3 and other components. Further, the sterilization device further comprises a power interface 61, wherein the power interface 61 is electrically connected to the blowing device 41 and the sterilization module 3 through the main control circuit board 8, and is configured to be connected to an external power source to supply power to the blowing device 41 and the sterilization module 3.

Preferably, as shown in FIG. 3 and FIG. 5, the sterilization device further comprises a wheel 51, a generator 52, and an energy storage module 54, wherein the wheel 51 is rotatably disposed at the opening 121 and configured to abut against the handrail 101 and rotate under the action of the handrail 101. That is, the wheel 51 can be driven to rotate by the handrail 101 during operation of the escalator 100. The generator 52 is in drive connection with the wheel 51 and configured to receive energy of motion from the wheel 51 and transfer it to electric energy. The energy storage module 54 is electrically connected to the generator 52 and configured to store the electric energy generated by the generator 52 and supplies power to the blowing device 41 and/or the sterilization module 3. In some embodiments, the energy storage module 54 may be storage battery. In other embodiments, the energy storage module 54 may be a capacitor. In this embodiment, the sterilization device is provided with the wheel 51, the generator 52 and the energy storage module 54. The wheel 51 is driven to rotate by means of the rotation of the handrail 101, the generator 52 is driven by the wheel 51 to generate electric energy which is stored in the energy storage module 54, and the electric energy is input to the main control circuit board 8 to be filtered and rectified to obtain pure and stable direct current, which is then supplied to the sterilization module 3. It can be understood that when the blowing device 41 is a DC fan or blower, the storage battery can directly supply power to the blowing device 41. When the blowing device 41 is an AC fan or blower, the sterilization device may further comprise a power conversion module for converting direct current of the storage battery into alternating current power to be supplied to the AC fan or blower. Further, the generator 52 may be anyone in the prior art without violating the concept of the application, and the invention has no limitation in this aspect.

Preferably, the sterilization device further comprises a power supply mode change-over switch 62, wherein the power supply mode change-over switch 62 is electrically connected to the main control circuit board 8 and is used for enabling one of the power interface 61 and the energy storage module 54 to supply power to the blowing device 41 and the sterilization module 3. When electric energy in the energy storage module 54 is sufficient to supply power to the sterilization module 3 for a period of time, the power supply mode change-over switch 62 can be operated to choose the energy storage module 54 to supply power to the sterilization module 3. When the electric energy in the energy storage module 54 is insufficient, the power supply mode change-over switch 62 can be operated to choose the external power source to supply power to the sterilization module 3, so as to ensure normal operation of the sterilization device.

Preferably, as shown in FIG. 1 and FIG. 4, the sterilization device further comprises an infrared detection module 71 disposed on the shell 1. When detecting that a passenger enters the escalator 100, the infrared detection module 71 sends a signal to the main control circuit board 8, and the main control circuit board 8 powers on the blowing device 41 and the sterilization module 3 to sterilize the handrail 101. When detecting that no passenger enters the escalator 100 within a period of time, the infrared detection module 71 sends a signal to the main control circuit board 8 again to enable the blowing device 41 and the sterilization module 3 to stop. In this way, the handrail 101 can be sterilized flexibly as required, and unnecessary energy consumption is reduced.

Preferably, in this embodiment, the infrared detection module 71 is disposed on a front side (a side opposite to the escalator 100) of the shell 1, such that the infrared detection module 71 can detect a passenger before the passenger enters the escalator, so as to enable the sterilization module 3 to start sterilization in advance. In other embodiments, the infrared detection module 71 may be disposed on the side, facing the entrance of the escalator 100, of the shell 1, such that the infrared detection module 71 can detect a passenger when the passengers reach the entrance of the escalator 100, and enables the sterilization module 3 to work at this moment. Optionally, the infrared detection module 71 may be any type in the prior art, and the specific structure and detection principle of the infrared detection module 71 will no longer be described here.

Further, as shown in FIG. 3, the sterilization device has a mandatory working mode in which the blowing device 41 and the sterilization module 3 are manually controlled to work, as well as an intelligent working mode in which the blowing device 41 and the sterilization module 3 are controlled by the infrared detection module 71 to work. The sterilization device further comprises a working mode change-over switch 63, wherein the working mode change-over switch 63 is electrically connected to the main control board 8 and is used for switching the mandatory working mode and the intelligent working mode. When the handrail 101 needs to be sterilized continuously, the sterilization device is switched into the mandatory working mode through the working mode change-over switch 63 to perform sterilization continuously. When the handrail 101 does not need to be continuously sterilized, the sterilization device is switched into the intelligent working mode through the working mode change-over switch 63 to perform sterilization when a passenger enters the escalator. In this way, the sterilization device can be used more flexibly.

Preferably, as shown in FIG. 1, FIG. 3 and FIG. 4, the sterilization device further comprises multiple indicator lights 73 electrically connected to the main control circuit board 8, and the multiple indicator lights 73 are used for displaying the current working mode and the current power supply mode of the sterilization device and whether sterilization is being performed by the sterilization device, such that management staff can observe the current operating state of the sterilization device more visually so as to manage the sterilization device.

Preferably, as shown in FIG. 1, the sterilization device further comprises a display screen 72, and the display screen 72 is disposed on the shell 1 and is used for displaying information such as advertisements and cue words, such that the sterilization device has more functions. It can be understood that electric energy generated by the generator 52 can also be supplied to the indicator lights 73 and the display screen 72 through the energy storage module 54, which can be set as required, and the invention has no limitation in this aspect.

Obviously, the above embodiments of the invention are merely illustrative ones used for clearly explaining the invention, and are not intended to limit the implementation of the invention. Those ordinarily skilled in the art can make changes to the specific implementation and application range of the invention according to the idea of the invention, and the contents in this specification should not be construed as limitations of the invention. Any modifications, equivalent substitutions and improvements made based on the spirit and principle of the invention should fall within the protection scope of the claims of the invention.

## Claims

1. A sterilization device, **characterized by** comprising:
a shell (1) having a side defining an opening (121) configured to face a handrail (101) of an escalator (100);
an air guide passage (2) disposed inside the shell (1), the air guide passage (2) comprising an air inlet (21) communicated with an ambient space outside the shell (1), and an air outlet (22) oriented to and communicated with the opening (121);
a sterilization module (3) comprising a plasma generator (31) disposed in the air guide passage (2), the plasma generator (31) being configured to ionize air and generate high-energy particles; and
a blowing device (41) configured to force air outside the shell (1) into the air guide passage (2) and blow ionized air and the high-energy particles to the opening (121) via the air outlet (22) of the air guide passage (2).

2. The sterilization device according to Claim 1, **characterized in that** the blowing device (41) is disposed in the air guide passage (2) and is located upstream of the plasma generator (31).

3. The sterilization device according to Claim 1, **characterized in that** the shell (1) is provided with an air inlet opening, a grating (13) is disposed at the air inlet opening, the ambient space outside the shell (1) and an inside space within the shell (1) are communicated with each other through the grating (13), and the air inlet (21) of the air guide passage (2) is communicated with the inside space within the shell (1).

4. The sterilization device according to Claim 3, **characterized in that** a filter module is disposed at the air inlet (21) of the air guide passage (2).

5. The sterilization device according to Claim 1, **characterized in that** the sterilization module (3) further comprises an ultraviolet sterilization lamp (32) disposed in the shell (1), and the ultraviolet sterilization lamp (32) faces the opening (121) and is configured to emit ultraviolet light to the handrail (101) through the opening (121).

6. The sterilization device according to any one of Claims 1-5, **characterized in that** the sterilization device further comprises:
a wheel (51) rotatably disposed at the opening (121) and configured to abut against the handrail (101) and be rotated under action of the handrail (101);
a generator (52) in drive connection with the wheel (51) and configured to receive energy of motion from the wheel (51) and transfer the energy of motion to electric energy; and
an energy storage module (54) configured to store the electric energy generated by the generator (52) and supply power to the blowing device (41) and/or the sterilization module (3).

7. The sterilization device according to Claim 6, **characterized by** further comprising a power interface (61), wherein the power interface (61) is electrically connected to the blowing device (41) and the sterilization module (3), and is configured to be connected to an external power source.

8. The sterilization device according to Claim 7, **characterized by** further comprising a power supply mode change-over switch (62), wherein the power supply mode change-over switch (62) is configured to select one of the power interface (61) and the energy storage module (54) to supply power to the blowing device (41) and the sterilization module (3).

9. The sterilization device according to any one of Claims 1-8, **characterized by** further comprising an infrared detection module (71) disposed on the shell (1), wherein the infrared detection module (71) is configured to enable the blowing device (41) and the sterilization module (3) to work when detecting that a passenger enters the escalator (100).

10. The sterilization device according to Claim 9, **characterized in that** the infrared detection module (71) is disposed at another side of the shell which is opposite to the side in which the opening (121) is defined.

11. The sterilization device according to Claim 9, **characterized in that** the sterilization device has a mandatory working mode in which the blowing device (41) and the sterilization module (3) are manually controlled to work, and an intelligent working mode in which the blowing device (41) and the sterilization module (3) are controlled by the infrared detection module (71) to work; and
the sterilization device further comprises a working mode change-over switch (63) configured to switch the mandatory working mode and the intelligent working mode.

12. The sterilization device according to any one of Claims 1-11, **characterized in that** the sterilization device further comprises a display screen (72) disposed on the shell (1).

13. The sterilization device according to any one of Claims 1-12, **characterized in that** the sterilization device further comprises one or more indicator lights (73) disposed on the shell (1).

14. The sterilization device according to any one of Claims 1-13, **characterized in that** the opening (121) is elongated and defines a lengthwise direction and a depth direction perpendicular to the lengthwise direction such that the handrail (101) of the escalator (100) can move in the opening (121) along the lengthwise direction, the lengthwise direction and the depth direction both being slanted relative to a supporting surface on which the sterilization device stands.

15. The sterilization device according to Claim 14, **characterized in that** the air outlet (22) has an orientation parallel to the depth direction of the opening (121).
